Europäisches Patentamt

**European Patent Office**

**Office européen des brevets**

(11) Numéro de publication: **0 443 956 B1**

(12) **FASCICULE DE BREVET EUROPEEN**

(45) Date de publication de fascicule du brevet: **31.08.94**

(51) Int. Cl.5: **C07C 69/68**, C07C 67/08, A61K 7/40, A61K 31/23

(21) Numéro de dépôt: **91400472.6**

(22) Date de dépôt: **21.02.91**

(54) **Acylats d'acide lactique, leurs sels, leur procédé de préparation et les compositions les renfermant.**

(30) Priorité: **22.02.90 FR 9002176**

(43) Date de publication de la demande:
**28.08.91 Bulletin 91/35**

(45) Mention de la délivrance du brevet:
**31.08.94 Bulletin 94/35**

(84) Etats contractants désignés:
**AT BE CH DE DK ES FR GB GR IT LI LU NL SE**

(56) Documents cités:
DE-A- 2 456 631
FR-A- 2 183 070
US-A- 3 933 825

(73) Titulaire: **ROUSSEL-UCLAF**
**35, Boulevard des Invalides**
**F-75007 Paris (FR)**

(72) Inventeur: **Noel, Hugues**
**28, rue du Général Lherillier**
**F-95120 Ermont (FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Rank Xerox (UK) Business Services
(3.10/3.09/3.3.3)

**Description**

La présente invention a pour objet des produits de formule (I) :

R-CO-[OCH(CH$_3$)-CO]$_n$-OH     (I)

dans laquelle R représente un radical alkényle renfermant 10 atomes de carbone et n représente les valeurs de 1 à 3, ainsi que leurs sels, lesdits produits de formule (I) étant sous toutes les formes isomères possibles, racémiques, énantiomères et diastéréoisomères.

De tels produits de formule (I) telle que définie ci-dessus portent le nom d'acylats d'acide lactique.

Dans les produits de formule (I) tels que définis ci-dessus, R représente un radical décényle, c'est-à-dire un radical alkényle renfermant 10 atomes de carbone et une double liaison pouvant se situer à un endroit quelconque sur la chaîne.

Un tel radical décényle peut ainsi représenter, par exemple, le radical 8-décényle, 7-décényle ou 6-décényle, mais représente, de préférence, le radical 9-décényle, le chiffre indiqué dans ces radicaux donnant la position du premier des deux atomes de carbone qui portent la double liaison, les atomes de carbone étant comptés à partir de l'atome de carbone lié au groupement -CO-.

Les sels des produits de formule (I) telle que définie ci-dessus peuvent être cités notamment les sels de métaux tels que l'aluminium, le cuivre, le magnésium ou le zinc, mais également les sels de sodium, de potassium, de triéthanolamine ou encore de diméthylaminoéthanol.

On peut citer, par exemple, les lactylates de zinc, de sodium ou de potassium.

La présente invention a particulièrement pour objet des produits de formule (I) telle que définie ci-dessus dans laquelle R représente le radical 9-décényle de formule :

CH$_2$ = CH(CH$_2$)$_8$-

et n représente les valeurs 1 ou 2, ainsi que leurs sels, sous toutes les formes isomères possibles racémiques, énantiomères et diastéréoisomères.

De tels acylats d'acide lactique correspondent donc à l'acide 10-undécénoyl 1-lactylique quand n est égal à 1 et à l'acide 10-undécénoyl 2-lactylique quand n est égal à 2.

La présente invention a tout particulièrement pour objet l'acide 10-undécénoyl 1-lactylique ainsi que ses sels, sous toutes les formes isomères possibles racémiques, énantiomères et diastéréoisomères.

La présente invention a encore pour objet un procédé de préparation des produits de formule (I) telle que définie ci-dessus, caractérisé en ce que l'on soumet un équivalent d'un produit de formule (II) :

R-COOH     (II)

dans laquelle R représente un radical alkényle renfermant 10 atomes de carbone, à l'action d'environ 1 à 3 équivalents d'acide lactique :

CH$_3$-CHOH-COOH

sous forme racémique ou optiquement active et, si désiré :
- traite le produit de formule (I) obtenu par une base minérale ou organique pour obtenir le sel correspondant,
- sépare les produits de formule (I) racémiques en leurs énantiomères ou diastéréoisomères.

Le produit de formule (II) est un acide undécylénique.

La réaction d'acylation du produit de formule (II) avec l'acide lactique peut être obtenue par contact direct, sans solvant, en présence d'un catalyseur tel que, par exemple, l'acide paratoluènesulfonique.

L'obtention des produits dans lesquels n varie de 1 à 3 peut être réalisée par la mise en présence d'une proportion relative variable du produit de formule (II) et d'acide lactique. Ainsi, on utilise une mole du produit de formule (II) pour un nombre n de moles d'acide lactique selon le produit de formule (I) à obtenir.

Le traitement des produits de formule (I) par une base minérale ou organique pour obtenir le sel correspondant ainsi que la séparation des produits de formule (I) racémiques en leurs énantiomères ou diastéréoisomères, peuvent être réalisés, selon les méthodes usuelles connues de l'homme de métier.

Or il vient d'être trouvé, et c'est un autre objet de la présente invention, que les acylats d'acide lactique ainsi que leurs sels ont une activité comédolytique importante.

Les comédons se forment dans les follicules pilosébacés par suite d'anomalies de la kératinisation et de la desquamation conduisant à une accumulation de matériel kératinisé qui forme un bouchon.

L'acné vulgaire et l'application régulière de produits cosmétiques mal adaptés ou renfermant des substances comédogènes sont des causes fréquentes de l'apparition de comédons.

L'utilisation de substances à effet kératolytique et sébosuppressive est connue depuis longtemps dans le traitement des comédons, notamment la vitamine A acide pour le soin de l'acné, l'acide azélaïque ou encore l'acide 13-cis-rétinoïque.

Les acylats d'acide lactique ont l'avantage d'être, en particulier, beaucoup plus stables que la vitamine A acide, et sont, de plus, très bien tolérés.

La présente invention concerne donc des compositions pharmaceutiques destinées notamment au traitement des comédons.

L'invention a ainsi pour objet de nouvelles compositions pharmaceutiques caractérisées en ce qu'elles renferment l'un au moins des produits de formule (I) telle que définie ci-dessus, ou l'un au moins de leurs sels, sous toutes leurs formes isomères possibles, racémiques, énantiomères et diastéréoisomères.

L'invention a plus précisément pour objet de nouvelles compositions pharmaceutiques telles que définies ci-dessus, caractérisées en ce qu'elles renferment l'un au moins des produits de formule (I) dans laquelle :

R représente le radical 9-décényle de formule :

$$CH_2 = CH(CH_2)_8 -$$

et n représente les valeurs 1 ou 2, ou l'un au moins de leurs sels.

L'invention a tout particulièrement pour objet de nouvelles compositions pharmaceutiques telles que définies ci-dessus caractérisées en ce qu'elles renferment l'acide 10-undécénoyl 1-lactylique ou l'un de ses sels.

L'invention a plus particulièrement pour objet de nouvelles compositions pharmaceutiques telles que définies ci-dessus caractérisées en ce qu'elles renferment entre 0,1 et 75 % et de préférence entre 5 % et 25 % d'acide 10-undécénoyl 1-lactylique ou l'un de ses sels.

En raison de leurs très intéressantes propriétés comédolytiques illustrées ci-aprés, les compositions pharmaceutiques telles que définies ci-dessus, objet de l'invention, peuvent être utilisées pour le soin des peaux acnéiques, des peaux grasses ou séborrhéiques et des peaux ayant réagi à l'application de substances comédogènes.

Les compositions pharmaceutiques selon l'invention peuvent se présenter sous toutes les formes utilisées en dermatologie, plus particulièrement de manière à pouvoir être administrées par voie topique.

De telles compositions pharmaceutiques peuvent être solides, liquides ou gazeuses, présentées sous forme de mousse ou d'aérosol ou encore de poudres compactées : parmi de telles présentations peuvent être citées par exemple, les solutions, émulsions, crèmes, pommades, poudres, laits, laits de toilette, gels aqueux ou anhydres, lotions, lotion anti-pelliculaire, shampooings ou bains moussants.

De telles présentations sont conditionnées, selon le cas, en pots ou en tubes, en flacon de verre ou de plastique ou éventuellement en flacon doseur ou encore en ampoules.

Les formes pharmaceutiques sont préparées selon les méthodes usuelles.

L'invention a particulièrement pour objet les compositions pharmaceutiques telles que définies ci-dessus caractérisées en ce qu'elles se présentent sous forme de préparations liquides ou solides à usage topique.

Pour chaque forme, on a recours à des excipients appropriés.

Ces excipients doivent avoir toutes les qualités habituellement requises. Dans le cas d'une administration locale, ils doivent être doués d'une grande affinité pour la peau, être parfaitement bien tolérés, stables, présenter une consistance adéquate permettant une utilisation facile et agréable.

A titre d'exemples d'excipients pouvant être utilisés, on peut citer notamment les hydrocarbures, les huiles de silicones, les triglycérides de synthèse, les cires végétales, animales ou minérales, les acides et les alcools gras, les esters d'acides gras ou d'alcool gras, les amides d'acide gras, les tensio-actifs non ioniques, les tensio-actifs anioniques, les polysaccharides gélifiants naturels ou synthétiques, la chitine désacétylée, les dérivés de cellulose, les dérivés de guar, les polyols, les polyalkylèneglycols, les charges minérales, les pigments organiques ou les laques de colorants organiques ainsi que d'autres excipients connus et couramment utilisés tels que, par exemple, des polymères de type carboxyvinylique, les polyéthylèneglycols, le propylène glycol, des triglycérides d'acides gras, des dérivés stéariques tel que, par exemple, le stéarate de glycérol, des alcools tels que, par exemple, les alcools stéaryliques, les alcools cétostéaryliques, l'alcool cétylique, l'éther cétylique polyoxyéthylène, des huiles végétales telles que l'huile

3

d'amande douce, des huiles minérales telles que l'huile de vaseline, la glycérine, des dérivés de la lanoline, du talc, des mouillants, des épaississants, des stabilisants, des émulsionnants, des conservateurs, des parfums, des colorants.

L'invention a également pour objet de nouvelles compositions cosmétiques destinées notamment au soin des comédons caractérisées en ce qu'elles renferment des produits de formule (I) telle que définie ci-dessus, ou l'un de leurs sels.

Les compositions cosmétiques peuvent être présentées comme des produits usuels des soins de la peau, par exemple sous forme de crèmes hydratantes, crèmes écran total, crèmes de jour, crèmes de nuit, masques ou comme des produits de maquillage, par exemple, fond de teint et crème teintée, ou comme des produits de démaquillage ou encore comme des produits d'hygiène.

Les compositions cosmétiques peuvent être préparées en utilisant les mêmes ingrédients que dans les compositions pharmaceutiques. Les doses peuvent être adaptées si nécessaire.

Les mêmes excipients que ceux indiqués ci-dessus pour la préparation des compositions pharmaceutiques et qui sont les excipients habituels des industries pharmaceutique et cosmétique peuvent être utilisés dans la préparation de telles compositions cosmétiques.

Des principes actifs ordinairement utilisés dans les produits de soin de la peau peuvent également entrer dans la préparation de telles compositions cosmétiques : parmi ces principes actifs ordinaires, peuvent être cités, par exemple, les agents hydratants tels que la glycérine, les sels d'acides aminés, le sorbitol, le glycol, le polyéthylène glycol, les filtres solaires, la dihydroxyacétone, l'oxyde de zinc, les agents anti-pelliculaires tel que la piroctone olamine, les éthanolamides d'acide undécylénique, les acylats de protéines hydrolysées y compris abiétate (acide terpénique du bois résineux) et undécylénate, les conservateurs fongicides et bactéricides tels que ceux listés, par exemple, dans l'annexe 6 de la directive européenne 76 768 CEE, le péroxyde de benzoyle, les extraits de plantes, les extraits d'organes d'animaux, les sérums.

L'invention concerne ainsi notamment l'application, à titre de produit cosmétique, des compositions telles que définies ci-dessus.

L'invention concerne tout particulièrement une méthode de traitement cosmétique des comédons caractérisée en ce que l'on utilise les compositions cosmétiques telles que définies ci-dessus.

Les différentes formes pharmaceutiques et cosmétiques mentionnées ci-dessus peuvent être obtenues selon les méthodes usuelles utilisées dans ces domaines.

Les produits de départ utilisés de formule (II) telle que définie ci-dessus et l'acide lactique sont des produits commerciaux, en particulier les produits de formule (II) sont vendus par la firme ATOCHEM.

Les exemples suivants illustrent l'invention sans toutefois la limiter.

**EXEMPLE 1** : **Acide 10-undécénoyl 2-lactylique**

On mélange, de préférence sous pression réduite, 400 grammes d'acide lactique à 90 % et 368,5 grammes d'acide 10-undécylénique : la réaction est réalisée en présence d'acide paratoluènesulfonique par chauffage pendant environ 10 heures à une température d'environ 150°C.

On obtient 674,5 grammes d'acides undécénoyl lactyliques, ce mélange étant principalement constitué d'acide 10-undécénoyl 2-lactylique.

**EXEMPLE 2** : **Acide 10-undécénoyl 1-lactylique**

On mélange 32,8 grammes du produit de l'exemple 1, 200 cm$^3$ d'eau distillée et 8 grammes d'hydroxyde de sodium : le mélange est maintenu pendant environ trois semaines à une température d'environ 25°C, le pH se stabilisant à 6,9.

Aprés addition d'acide chlorhydrique jusqu'à pH = 1, on extrait par environ 100 cm$^3$ de chloroforme, évapore le solvant et isole le produit attendu.

**EXEMPLE 3** : **10-undécénoyl 2-lactylate de zinc**

On broie pendant environ 3 jours consécutifs 32,8 grammes du produit de l'exemple 1 et 4 grammes d'oxyde de zinc puis séche en dessicateur sur silice anhydre : en une semaine environ, une pâte homogène est obtenue.

4

**EXEMPLE 4** : **10-undécénoyl 1-lactylate de zinc**

On ajoute 120 cm$^3$ d'une solution aqueuse 1N de sulfate de zinc à la solution du produit de l'exemple 2, obtenue après stabilisation du pH à 6,9. Le précipité huileux qui se forme est lavé quatre fois par environ 50 cm$^3$ d'eau distillée puis séché selon le procédé décrit à l'exemple 3.

**EXEMPLE 5** : **Fond de teint.**

| | |
|---|---|
| Monostéarate de pentaérythritol | 6,5 |
| Monoundécylénate de glycérol | 1,0 |
| Sitostérol | 0,8 |
| Monostéarate de sorbitan | 1,0 |
| Huile de macadamia | 1,2 |
| Huile d'onagre | 2,0 |
| Perhydrosqualene | 0,5 |
| Stéaryl diméthyl siloxane | 2,0 |
| Cyclosiloxane | 5,0 |
| Benzoate de lauryle | 5,0 |
| Nonylphénol éthoxylé | 1,0 |
| Acide stéarique éthoxylé | 2,2 |
| Abiétate de protéines hydrolysées et de potassium | 3,0 |
| Oxyde de zinc | 3,0 |
| 10-undécénoyl 1-lactylate de zinc | 7,0 |
| Oxyde de titane | 3,5 |
| Talc | 2 |
| Silice | 1,0 |
| Oxyde de fer | 3,0 |
| Butanediol-1,3 | 5,0 |
| Conservateur | 0,3 |
| Parfum | 0,2 |
| Eau déminéralisée | qsp 100 |

**EXEMPLE** 6 : **Crème hydratante.**

| | |
|---|---|
| Sitostérol | 1,0 |
| Huile de macadamia | 3,0 |
| Laurate myristate d'éthyl héxyle | 9,0 |
| Alcool cétylique | 0,7 |
| Décaméthyl cyclopentasiloxane | 6,0 |
| Paraméthoxycinnamate d'éthylhéxyle | 2,0 |
| Gamma orizanol | 0,5 |
| Isohéxadécane | 12,0 |
| Monostéarate de pentaérythritol | 8,0 |
| Glucosamine chlorhydrate | 0,2 |
| Acide lactique | 0,5 |
| Pyrolidone carboxylate de sodium | 0,5 |
| 10-undécénoyl 1-lactylate de sodium | 7,0 |
| Glycérine | 2,0 |
| | |
| Conservateur | 0,3 |
| Parfum | 0,3 |
| Eau déminéralisée | qsp 100 |

**EXEMPLE 7** : Lait de toilette.

| | |
|---|---|
| Eau déminéralisée | qsp 100 |
| Acide citrique | 0,5 |
| Acide stéarique éthoxylé | 2,5 |
| Monostéarate de glycérol | 2,5 |
| Polysorbate 60 | 2,0 |
| Lactate de monoglycérides | 2,6 |
| Caprate caprylate de propanediol | 2,4 |
| Palmitate d'éthylhéxyle | 8,0 |
| Oléate de sorbitan | 2,0 |
| Monostéarate de sorbitan | 1,0 |
| Extrait de millepertuis | 3,0 |
| Eau de sureau | 4,0 |
| Parfum | 0,3 |
| Conservateur | 0,3 |
| 10-undécénoyl 1-lactylate de sodium | 3,0 |

**EXEMPLE 8** : Lotion antipelliculaire.

| | |
|---|---|
| 10-undécénoyl 1-lactylate de potassium | 1,0 |
| Alcool laurique éthoxylé | 1,0 |
| Alcool octyl-2-dodécylique éthoxylé | 0,9 |
| Monoéthanolamide d'acide undécylénique | 0,5 |
| OCTOPIROX $^{R}$ | 0,4 |
| Carbomer 940 | 0,15 |
| Acide citrique | 0,10 |
| Conservateur | 0,30 |
| Parfum | 0,10 |
| Eau déminéralisée | qsp 100 |

,

**EXEMPLE 9** : **Ecran total.**

| | |
|---|---|
| Hectorite | 1,0 |
| Butanediol 1,3 | 3,0 |
| Extrait de chardon marie | 3,0 |
| Acide phénylbenzimidazolsulfonique | 2,0 |
| L lysine 50 % | 2,2 |
| 10-undécénoyl 1-lactylate de zinc | 10,0 |
| Monostéarate de propylène glycol | 1,8 |

| | |
|---|---|
| Acide stéarique | 3,0 |
| Sitostérol | 2,0 |
| Stéaroyl N méthyl taurate de sodium | 1,0 |
| Huile d'onagre | 1,5 |
| Beurre de karité | 2,0 |
| Huile de carthame hybride | 1,0 |
| Tripalmitate stéarate de glycérol | 8,0 |
| Huile de coprah hydrogénée | 5,0 |
| Cyclosiloxane | 2,0 |
| Phénylméthylsiloxane | 1,0 |
| Paraméthoxycinnamate d'éthylhéxyle | 7,5 |
| Méthoxy butyl dibenzoyl méthane | 2,0 |
| DL alpha-tocophérol | 0,1 |
| Conservateur | 0,3 |
| Parfum | 0,3 |
| Eau déminéralisée | qsp 100 |

**Test d'activité comédolytique du produit de l'exemple 6.**

L'animal choisi pour le test d'activité comédolytique est la souris Hairless Rhino (hr rh), de sexe femelle, ce choix étant dû au fait que la peau d'un tel animal présente une grande densité de comédons de large diamètre et d'orifice étroit.

L'application d'agents comédolytiques sur la peau de l'animal provoque l'ouverture de l'orifice du comédon, la libération du matériel corné et du sébum qu'il contient.

Deux lots sont constitués, chacun des lots comportant 6 souris âgées de 6 semaines au début de l'essai et pesant en moyenne 18 grammes chacune.

Le premier lot est constitué de souris traitées avec de l'eau distillée (lot témoin négatif), le second lot est constitué de souris traitées avec le produit de l'exemple 6, un tel traitement consistant en une application topique du produit étudié sur la zone interscapulaire à la dose de 0,02 ml, 5 jours sur 7 pendant 21 jours.

Les animaux sont sacrifiés à l'issue des trois semaines de traitement, 24 heures après la dernière application.

Des biopsies de la peau sont alors prélevées au niveau des zones traitées des animaux et à partir de ces biopsies, des coupes sont préparées, en vue d'une étude morphométrique classique réalisée selon les méthodes connues de l'homme de métier.

Les paramètres suivants sont mesurés :

diamètre de l'ouverture de comédon à la surface soit d

diamètre du comédon                                    soit D

profil comédonien                                      soit R = d/D

Le rapport R = d/D permet de quantifier l'action des agents comédolytiques.

Le pourcentage d'inhibition des comédons est calculé pour le produit de l'exemple 6 par rapport au témoin négatif, soit le rapport suivant :

$$\% \text{ inhibition} = \frac{(R \text{ produit} - R \text{ témoin négatif}) \times 100}{R \text{ témoin négatif}}$$

Le résultat obtenu est de 121,9, montrant ainsi l'efficacité du produit étudié.

**Revendications**

**Revendications pour les Etats contractants suivants : AT, BE, CH, DE, DK, FR, GB, IT, LI, LU, NL, SE**

1. Produits de formule (I) :

   $R\text{-}CO\text{-}[OCH(CH_3)\text{-}CO]_n\text{-}OH$     (I)

   dans laquelle R représente un radical alkényle renfermant 10 atomes de carbone et n représente les valeurs de 1 à 3, ainsi que leurs sels, lesdits produits de formule (I) étant sous toutes les formes isomères possibles, racémiques, énantiomères et diastéréoisomères.

2. Produits de formule (I) telle que définie à la revendication 1 dans laquelle R représente le radical 9-décényle de formule :

   $CH_2 = CH(CH_2)_8\text{-}$

   et n représente les valeurs 1 ou 2, ainsi que leurs sels, sous toutes les formes isomères possibles, racémiques et énantiomères et diastéréoisomères.

3. L'acide 10-undécénoyl 1-lactylique ainsi que ses sels, sous toutes les formes isomères possibles, racémiques, énantiomères et diastéréoisomères.

4. Procédé de préparation des produits de formule (I) telle que définie à l'une quelconque des revendications 1 à 3, caractérisé en ce que l'on soumet un équivalent d'un produit de formule (II) :

   $R\text{-}COOH$     (II)

   dans laquelle R représente un radical alkényle renfermant 10 atomes de carbone, à l'action d'environ 1 à 3 équivalents d'acide lactique :

   $CH_3\text{-}CHOH\text{-}COOH$

   sous forme racémique ou optiquement active et, si désiré :
   - traite le produit de formule (I) obtenu par une base minérale ou organique pour obtenir le sel correspondant,

- sépare les produits de formule (I) racémiques en leurs énantiomères ou diastéréoisoméres.

**5.** Compositions pharmaceutiques caractérisées en ce qu'elles renferment l'un au moins des produits de formule (I) telle que définie à la revendication 1, ou l'un au moins de leurs sels sous toutes les formes isomères possibles, racémiques, énantiomères et diastéréoisomères.

**6.** Compositions pharmaceutiques telles que définies à la revendication 5 caractérisées en ce qu'elles renferment l'un au moins des produits de formule (I) dans laquelle R représente le radical 9-décényle de formule :

$$CH_2 = CH(CH_2)_8 -$$

et n représente les valeurs 1 ou 2, ou l'un au moins de leurs sels.

**7.** Compositions pharmaceutiques telles que définies à la revendication 5 ou 6 caractérisées en ce qu'elles renferment de l'acide 10-undécénoyl 1-lactylique ou l'un de ses sels.

**8.** Compositions pharmaceutiques telles que définies à l'une quelconque des revendications 5 à 7 caractérisées en ce qu'elles renferment entre 0,1 et 75 % et de préférence entre 5 % et 25 % d'acide 10-undécénoyl 1-lactylique ou l'un de ses sels.

**9.** Compositions pharmaceutiques telles que définies à l'une quelconque des revendications 5 à 8 caractérisées en ce qu'elles se présentent sous forme de préparations liquides ou solides à usage topique.

**10.** Compositions cosmétiques destinées notamment au soin des comédons caractérisées en ce qu'elles renferment des produits de formule (I) telle que définie à l'une quelconque des revendications 1 à 3, ou l'un de leurs sels.

**11.** Application à titre de produit cosmétique des compositions telles que définies à la revendication 10.

**12.** Méthode de traitement cosmétique des comédons caractérisée en ce que l'on utilise les compositions cosmétiques telles que définies à la revendication 10.

**Revendications pour l'Etat contractant suivant : ES**

**1.** Procédé de préparation des produits de formule (I) :

$$R-CO-[OCH(CH_3)-CO]_n-OH \qquad (I)$$

dans laquelle R représente un radical alkényle renfermant 10 atomes de carbone et n représente les valeurs de 1 à 3, ainsi que leurs sels, lesdits produits de formule (I) étant sous toutes les formes isomères possibles, racémiques, énantiomères et diastéréoisomères, caractérisé en ce que l'on soumet un équivalent d'un produit de formule (II) :

$$R-COOH \qquad (II)$$

dans laquelle R représente un radical alkényle renfermant 10 atomes de carbone, à l'action d'environ 1 à 3 équivalents d'acide lactique :

$$CH_3-CHOH-COOH$$

sous forme racémique ou optiquement active et, si désiré :
- traite le produit de formule (I) obtenu par une base minérale ou organique pour obtenir le sel correspondant,
- sépare les produits de formule (I) racémiques en leurs énantiomères ou diastéréoisomères.

2. Procédé selon la revendication 1, caractérisé en ce que l'on utilise au départ 1 mole d'un produit de formule (II) dans laquelle R représente le radical 9-décényle de formule :

$CH_2 = CH(CH_2)_8-$

et 1 ou 2 moles d'acide lactique.

3. Procédé selon la revendication 1 ou 2, caractérisé en ce que l'on utilise au départ 1 mole d'acide de formule $CH_2=CH(CH_2)_8-COOH$ et 1 mole d'acide lactique.

4. Procédé de préparation de compositions cosmétiques destinées au traitement cosmétique des comédons, caractérisé en ce que l'on met à titre de principe actif l'un au moins des composés de formule (I) telle que définie à l'une quelconque des revendications 1 à 3 ou l'un au moins de leurs sels, sous toutes les formes isomères possibles, racémiques, énantiomères ou diastéréoisomères, sous une forme destinée à cet usage.

5. Application à titre de produit cosmétique des compositions telles que définies à la revendication 4.

6. Méthode de traitement cosmétique des comédons caractérisée en ce que l'on utilise les compositions cosmétiques telles que définies à la revendication 4.

**Revendications pour l'Etat contractant suivant : GR**

1. Procédé de préparation des produits de formule (I) :

$R-CO-[OCH(CH_3)-CO]_n-OH$     (I)

dans laquelle R représente un radical alkényle renfermant 10 atomes de carbone et n représente les valeurs de 1 à 3, ainsi que leurs sels, lesdits produits de formule (I) étant sous toutes les formes isomères possibles, racémiques, énantiomères et diastéréoisomères, caractérisé en ce que l'on soumet un équivalent d'un produit de formule (II) :

$R-COOH$     (II)

dans laquelle R représente un radical alkényle renfermant 10 atomes de carbone, à l'action d'environ 1 à 3 équivalents d'acide lactique :

$CH_3-CHOH-COOH$

sous forme racémique ou optiquement active et, si désiré :
- traite le produit de formule (I) obtenu par une base minérale ou organique pour obtenir le sel correspondant,
- sépare les produits de formule (I) racémiques en leurs énantiomères ou diastéréoisomères.

2. Procédé selon la revendication 1, caractérisé en ce que l'on utilise au départ 1 mole d'un produit de formule (II) dans laquelle R représente le radical 9-décényle de formule :

$CH_2 = CH(CH_2)_8-$

et 1 ou 2 moles d'acide lactique.

3. Procédé selon la revendication 1 ou 2, caractérisé en ce que l'on prépare l'acide 10-undécénoyl 1-lactylique ainsi que ses sels, sous toutes les formes isomères possibles, racémiques, énantiomères et diastéréoisomères.

4. Procédé de préparation de compositions pharmaceutiques, caractérisé en ce que l'on met à titre de principe actif l'un au moins des composés de formule (I) ou de leurs sels, sous toutes les formes isomères possibles racémiques, énantiomères ou diastéréoisomères, pharmaceutiquement acceptables, sous une forme destinée à cet usage.

5. Procédé selon la revendication 4, caractérisé en ce que l'on met à titre de principe actif l'un au moins des composés de formule (I) tel qu'obtenu à la revendication 2 ou de leurs sels, sous toutes les formes isomères possibles racémiques, énantiomères ou diastéréoisomères, pharmaceutiquement acceptables, sous une forme destinée à cet usage.

6. Procédé selon la revendication 4 ou 5, caractérisé en ce que l'on met à titre de principe actif l'acide 10-undécénoyl 1-lactylique ou l'un de ses sels.

7. Procédé selon l'une quelconque des revendications 4 à 6, caractérisé en ce que l'on met à titre de principe actif entre 0,1 et 75 % et de préférence entre 5 % et 25 % d'acide 10-undécénoyl 1-lactylique ou l'un de ses sels.

8. Procédé selon l'une quelconque des revendications 4 à 7, caractérisé en ce que l'on met le principe actif dans une préparation sous forme liquide ou solide à usage topique.

9. Compositions cosmétiques destinées notamment au soin des comédons caractérisées en ce qu'elles renferment des produits de formule (I) telle que définie à l'une quelconque des revendications 1 à 3, ou l'un de leurs sels.

10. Application à titre de produit cosmétique des compositions telles que définies à la revendication 9.

11. Méthode de traitement cosmétique des comédons caractérisée en ce que l'on utilise les compositions cosmétiques telles que définies à la revendication 9.

12. Procédé de préparation de compositions cosmétiques destinées au traitement cosmétique des comédons, caractérisé en ce que l'on met à titre de principe actif l'un au moins des composés de formule (I) telle que définie à l'une quelconque des revendications 1 à 3 ou l'un au moins de leurs sels, sous toutes les formes isomères possibles, racémiques, énantiomères ou diastéréoisomères, sous une forme destinée à cet usage.

**Claims**
**Claims for the following Contracting States : AT, BE, CH, DE, DK, FR, GB, IT, LI, LU, NL, SE**

1. Products of formula (I):

   $$R\text{-}CO\text{-}[OCH(CH_3)\text{-}CO]_n\text{-}OH \qquad (I)$$

   in which R represents an alkenyl radical containing 10 carbon atoms and n represents the values 1 to 3, as well as their salts, said products of formula (I) being in all the possible racemic, enantiomeric and diastereoisomeric isomer forms.

2. Products of formula (I) as defined in claim 1 in which R represents the 9-decenyl radical of formula:

   $$CH_2 = CH(CH_2)_8\text{-}$$

   and n represents the values 1 or 2, as well as their salts, in all the possible racemic and enantiomeric and diastereoisomeric isomer forms.

3. 10-undecenoyl 1-lactylic acid as well as its salts, in all the possible racemic, enantiomeric and diastereoisomeric isomer forms.

4. Preparation process for products of formula (I) as defined in any one of claims 1 to 3, characterized in that an equivalent of a product of formula (II):

   $$R\text{-}COOH \qquad (II)$$

EP 0 443 956 B1

in which R represents an alkenyl radical containing 10 carbon atoms, is subjected to the action of about 1 to 3 equivalents of lactic acid:

$CH_3$-CHOH-COOH

in racemic or optically active form and, if desired:
- the product of formula (I) obtained is treated with a mineral or organic base in order to obtain the corresponding salt,
- the racemic products of formula (I) are separated into their enantiomers or diastereoisomers.

5. Pharmaceutical compositions characterized in that they contain at least one of the products of formula (I) as defined in claim 1, or at least one of their salts, in all the possible racemic, enantiomeric and diastereoisomeric isomer forms.

6. Pharmaceutical compositions as defined in claim 5 characterized in that they contain at least one of the products of formula (I) in which R represents the 9-decenyl radical of formula:

$CH_2 = CH(CH_2)_8 -$

and n represents the values 1 or 2, or at least one of their salts.

7. Pharmaceutical compositions as defined in claim 5 or 6 characterized in that they contain 10-undecenoyl 1-lactylic acid or one of its salts.

8. Pharmaceutical compositions as defined in any one of claims 5 to 7 characterized in that they contain between 0.1 and 75% and preferably between 5% and 25% of 10-undecenoyl 1-lactylic acid or one of its salts.

9. Pharmaceutical compositions as defined in any one of claims 5 to 8 characterized in that they are presented in the form of liquid or solid preparations for topical use.

10. Cosmetic compositions intended in particular for treating blackheads characterized in that they contain products of formula (I) as defined in any one of claims 1 to 3, or one of their salts.

11. Use as a cosmetic product of the compositions as defined in claim 10.

12. Cosmetic treatment method for blackheads characterized in that the cosmetic compositions as defined in claim 10 are used.

**Claims for the following Contracting State : ES**

1. Preparation process for products of formula (I):

R-CO-[OCH($CH_3$)-CO]$_n$-OH      (I)

in which R represents an alkenyl radical containing 10 carbon atoms and n represents the values 1 to 3, as well as their salts, said products of formula (I) being in all the possible racemic, enantiomeric and diastereoisomeric isomer forms, characterized in that an equivalent of a product of formula (II):

R-COOH      (II)

in which R represents an alkenyl radical containing 10 carbon atoms, is subjected to the action of about 1 to 3 equivalents of lactic acid:

$CH_3$-CHOH-COOH

in racemic or optically active form and, if desired:

13

- the product of formula (I) obtained is treated with a mineral or organic base in order to obtain the corresponding salt,
- the racemic products of formula (I) are separated into their enantiomers or diastereoisomers.

2. Process according to claim 1, characterized in that at the start 1 mole of a product of formula (II) in which R represents the 9-decenyl radical of formula:

$$CH_2 = CH(CH_2)_8-$$

and 1 or 2 moles of lactic acid, are used.

3. Process according to claim 1 or 2, characterized in that 1 mole of acid of formula $CH_2 = CH(CH_2)_8-COOH$ and 1 mole of lactic acid are used at the start.

4. Preparation process for cosmetic compositions intended for the cosmetic treatment of blackheads, characterized in that at least one of the compounds of formula (I) as defined in any one of claims 1 to 3 or at least one of their salts, in all the possible racemic, enantiomeric or diastereoisomeric isomer forms, is used as active ingredient in a form intended for this use.

5. Use as a cosmetic product of the compositions as defined in claim 4.

6. Cosmetic treatment method for blackheads characterized in that the cosmetic compositions as defined in claim 4 are used.

**Claims for the following Contracting State : GR**

1. Preparation process for products of formula (I):

$$R-CO-[OCH(CH_3)-CO]_n-OH \qquad (I)$$

in which R represents an alkenyl radical containing 10 carbon atoms and n represents the values 1 to 3, as well as their salts, said products of formula (I) being in all the possible racemic, enantiomeric and diastereoisomeric isomer forms, characterized in that an equivalent of a product of formula (II):

$$R-COOH \qquad (II)$$

in which R represents an alkenyl radical containing 10 carbon atoms, is subjected to the action of about 1 to 3 equivalents of lactic acid:

$$CH_3-CHOH-COOH$$

in racemic or optically active form and, if desired:
- the product of formula (I) obtained is treated with a mineral or organic base in order to obtain the corresponding salt,
- the racemic products of formula (I) are separated into their enantiomers or diastereoisomers.

2. Process according to claim 1, characterized in that at the start 1 mole of a product of formula (II) in which R represents the 9-decenyl radical of formula:

$$CH_2 = CH(CH_2)_8-$$

and 1 or 2 moles of lactic acid, are used.

3. Process according to claim 1 or 2, characterized in that 10-undecenoyl 1-lactylic acid is prepared, as well as its salts, in all the possible racemic, enantiomeric and diastereoisomeric isomer forms.

4. Preparation process for pharmaceutical compositions, characterized in that at least one of the compounds of formula (I) or one of their pharmaceutically acceptable salts, in all the possible racemic, enantiomeric and diastereoisomeric isomer forms, is used as active ingredient in a form intended for this use.

5. Process according to claim 4, characterized in that at least one of the compounds of formula (I) as obtained in claim 2 or one of their pharmaceutically acceptable salts, in all the possible racemic, enantiomeric or diastereoisomeric isomer forms, is used as active ingredient in a form intended for this use.

6. Process according to claim 4 or 5, characterized in that 10-undecenoyl 1-lactylic acid or one of its salts is used as active ingredient.

7. Process according to any one of claims 4 to 6, characterized in that between 0.1 and 75% and preferably between 5% and 25% of 10-undecenoyl 1-lactylic acid or one of its salts is used as active ingredient.

8. Process according to any one of claims 4 to 7, characterized in that the active ingredient is used in a preparation in liquid or solid form for topical use.

9. Cosmetic compositions intended in particular for the treatment of blackheads characterized in that they contain products of formula (I) as defined in any one of claims 1 to 3, or one of their salts.

10. Use as a cosmetic product of the compositions as defined in claim 9.

11. Cosmetic treatment method for blackheads characterized in that the cosmetic compositions as defined in claim 9 are used.

12. Preparation process for cosmetic compositions intended for the cosmetic treatment of blackheads, characterized in that at least one of the compounds of formula (I) as defined in any one of claims 1 to 3 or at least one of their salts, in all the possible racemic, enantiomeric or diastereoisomeric isomer forms, is used as active ingredient in a form intended for this use.

**Patentansprüche**
**Patentansprüche für folgende Vertragsstaaten : AT, BE, CH, DE, DK, FR, GB, IT, LI, LU, NL, SE**

1. Produkte der Formel (I)

$R-CO-[OCH(CH_3)-CO]_n-OH$

in der R einen Alkenylrest mit 10 Kohlenstoffatomen darstellt und n die Werte 1 bis 3 besitzt, sowie ihre Salze, wobei die genannten Produkte der Formel (I) in allen möglichen isomeren, racemischen, enantiomeren und diastereoisomeren Formen vorliegen können.

2. Produkte der Formel (I) wie in Anspruch 1 definiert, worin R den 9-Decenyl-Rest der Formel

$CH_2 = CH(CH_2)_8 -$

darstellt und n die Werte 1 oder 2 besitzt, sowie ihre Salze, in allen möglichen isomeren, racemischen, enantiomeren und diastereoisomeren Formen.

3. 10-Undecenoyl-1-lactylsäure sowie ihre Salze, in allen möglichen isomeren, racemischen, enantiomeren und diastereoisomeren Formen.

4. Verfahren zur Herstellung der Produkte der Formel (I) wie in irgendeinem der Ansprüche 1 bis 3 definiert, dadurch gekennzeichnet, daß man ein Äquivalent eines Produktes der Formel (II)

$R-COOH$ (II)

in der R einen Alkenylrest mit 10 Kohlenstoffatomen darstellt, der Einwirkung von etwa 1 bis 3

Äquivalenten Milchsäure

$CH_3$-CHOH-COOH

in racemischer oder optisch aktiver Form unterzieht und, wenn gewünscht:
- das erhaltene Produkt der Formel (I) mit einer Mineralbase oder organischen Base behandelt, um das entsprechende Salz zu erhalten,
- die racemischen Produkte der Formel (I) in ihre Enantiomeren oder Diastereoisomeren auftrennt.

5. Pharmazeutische Zusammensetzungen, dadurch gekennzeichnet, daß sie mindestens eines der Produkte der Formel (I) wie in Anspruch 1 definiert oder mindestens eines ihrer Salze umfassen, in allen möglichen isomeren, racemischen, enantiomeren und diastereoisomeren Formen.

6. Pharmazeutische Zusammensetzungen wie in Anspruch 5 definiert, dadurch gekennzeichnet, daß sie mindestens eines der Produkte der Formel (I), in der R den 9-Decenyl-Rest der Formel

$CH_2 = CH(CH_2)_8 -$

darstellt und n die Werte 1 oder 2 besitzt oder mindestens eines ihrer Salze umfassen.

7. Pharmazeutische Zusammensetzungen wie in Anspruch 5 oder 6 definiert, dadurch gekennzeichnet, daß sie 10-Undecenoyl-1-lactylsäure oder eines ihrer Salze umfassen.

8. Pharmazeutische Zusammensetzungen wie in irgendeinem der Ansprüche 5 bis 7 definiert, dadurch gekennzeichnet, daß sie zwischen 0,1 und 75 % und vorzugsweise zwischen 5 und 25 % 10-Undecenoyl-1-lactylsäure oder eines ihrer Salze umfassen.

9. Pharmazeutische Zusammensetzungen wie in irgendeinem der Ansprüche 5 bis 8 definiert, dadurch gekennzeichnet, daß sie in Form von flüssigen oder festen Präparaten für die topische Anwendung vorliegen.

10. Kosmetische Zusammensetzungen, insbesondere vorgesehen zur Behandlung von Comedonen, dadurch gekennzeichnet, daß sie Produkte der Formel (I) wie in irgendeinem der Ansprüche 1 bis 3 definiert oder eines ihrer Salze umfassen.

11. Anwendung der Zusammensetzungen wie in Anspruch 10 definiert als kosmetisches Produkt.

12. Methode zur kosmetischen Behandlung von Comedonen, dadurch gekennzeichnet, daß man die kosmetischen Zusammensetzungen wie in Anspruch 10 definiert verwendet.

**Patentansprüche für folgenden Vertragsstaat : ES**

1. Verfahren zur Herstellung von Produkten der Formel (I)

R-CO-[OCH($CH_3$)-CO]$_n$-OH

in der R einen Alkenylrest mit 10 Kohlenstoffatomen darstellt und n die Werte 1 bis 3 besitzt, sowie ihre Salze, wobei die genannten Produkte der Formel (I) in allen möglichen isomeren, racemischen, enantiomeren und diastereoisomeren Formen vorliegen können, dadurch gekennzeichnet, daß man ein Äquivalent eines Produktes der Formel (II)

R-COOH      (II)

in der R einen Alkenylrest mit 10 Kohlenstoffatomen darstellt, der Einwirkung von etwa 1 bis 3 Äquivalenten Milchsäure

$CH_3$-CHOH-COOH

in racemischer oder optisch aktiver Form unterzieht und, wenn gewünscht:
- das erhaltene Produkt der Formel (I) mit einer Mineralbase oder organischen Base behandelt, um das entsprechende Salz zu erhalten,
- die racemischen Produkte der Formel (I) in ihre Enantiomeren oder Diastereoisomeren auftrennt.

2. Verfahren nach Anspruch 1, dadurch gekennzeichnet, daß man als Ausgangsprodukt 1 Mol eines Produktes der Formel (II), in der R den 9-Decenyl-Rest der Formel

$$CH_2 = CH(CH_2)_8 -$$

darstellt, und 1 oder 2 Mole Milchsäure verwendet.

3. Verfahren nach Anspruch 1 oder 2, dadurch gekennzeichnet, daß man als Ausgangsprodukt 1 Mol Säure der Formel

$$CH_2 = CH(CH_2)_8 -COOH$$

und 1 Mol Milchsäure verwendet.

4. Verfahren zur Herstellung von kosmetischen Zusammensetzungen, die für die kosmetische Behandlung von Comedonen vorgesehen sind, dadurch gekennzeichnet, daß man als Wirkstoff mindestens eine der Verbindungen der Formel (I) wie in irgendeinem der Ansprüche 1 bis 3 definiert oder mindestens eines ihrer Salze, in allen möglichen isomeren, racemischen, enantiomeren und diastereoisomeren Formen, in eine für diese Verwendung bestimmte Form bringt.

5. Anwendung der Zusammensetzungen wie in Anspruch 4 definiert als kosmetisches Produkt.

6. Methode zur kosmetischen Behandlung von Comedonen, dadurch gekennzeichnet, daß man die kosmetischen Zusammensetzungen wie in Anspruch 4 definiert verwendet.

**Patentansprüche für folgenden Vertragsstaat : GR**

1. Verfahren zur Herstellung von Produkten der Formel (I)

$$R-CO-[OCH(CH_3)-CO]_n-OH$$

in der R einen Alkenylrest mit 10 Kohlenstoffatomen darstellt und n die Werte 1 bis 3 besitzt, sowie ihre Salze, wobei die genannten Produkte der Formel (I) in allen möglichen isomeren, racemischen, enantiomeren und diastereoisomeren Formen vorliegen können, dadurch gekennzeichnet, daß man ein Äquivalent eines Produktes der Formel (II)

$$R-COOH \qquad (II)$$

in der R einen Alkenylrest mit 10 Kohlenstoffatomen darstellt, der Einwirkung von etwa 1 bis 3 Äquivalenten Milchsäure

$$CH_3-CHOH-COOH$$

in racemischer oder optisch aktiver Form unterzieht und, wenn gewünscht:
- das erhaltene Produkt der Formel (I) mit einer Mineralbase oder organischen Base behandelt, um das entsprechende Salz zu erhalten,
- die racemischen Produkte der Formel (I) in ihre Enantiomeren oder Diastereoisomeren auftrennt.

2. Verfahren nach Anspruch 1, dadurch gekennzeichnet, daß man als Ausgangsprodukt 1 Mol eines Produktes der Formel (II), in der R den 9-Decenyl-Rest der Formel

$$CH_2 = CH(CH_2)_8 -$$

darstellt, und 1 oder 2 Mole Milchsäure verwendet.

3. Verfahren nach Anspruch 1 oder 2, dadurch gekennzeichnet, daß man 10-Undecenoyl-1-lactylsäure sowie ihre Salze herstellt, in allen möglichen isomeren, racemischen, enantiomeren und diastereoisomeren Formen.

4. Verfahren zur Herstellung von pharmazeutischen Zusammensetzungen, dadurch gekennzeichnet, daß man als Wirkstoff mindestens eine der Verbindungen der Formel (I) oder ihre Salze, in allen möglichen pharmazeutisch akzeptablen, isomeren, racemischen, enantiomeren und diastereoisomeren Formen, in eine für diese Verwendung bestimmte Form bringt.

5. Verfahren nach Anspruch 4, dadurch gekennzeichnet, daß man als Wirkstoff mindestens eine der Verbindungen der Formel (I), wie in Anspruch 2 erhalten oder ihre Salze, in allen möglichen pharmazeutisch akzeptablen, isomeren, racemischen, enantiomeren und diastereoisomeren Formen, in eine für diese Verwendung bestimmte Form bringt.

6. Verfahren nach Anspruch 4 oder 5, dadurch gekennzeichnet, daß man als Wirkstoff 10-Undecenoyl-1-lactylsäure oder eines ihrer Salze verwendet.

7. Verfahren nach irgendeinem der Ansprüche 4 bis 6, dadurch gekennzeichnet, daß man als Wirkstoff zwischen 0,1 und 75 % und vorzugsweise zwischen 5 und 25 % 10-Undecenoyl-1-lactylsäure oder eines ihrer Salze verwendet.

8. Verfahren nach irgendeinem der Ansprüche 4 bis 7, dadurch gekennzeichnet, daß man den Wirkstoff in ein Präparat mit flüssiger oder fester Form für die topische Anwendung einbringt.

9. Kosmetische Zusammensetzungen, insbesondere vorgesehen zur Behandlung von Comedonen, dadurch gekennzeichnet, daß sie Produkte der Formel (I) wie in irgendeinem der Ansprüche 1 bis 3 definiert oder eines ihrer Salze umfassen.

10. Anwendung der Zusammensetzungen wie in Anspruch 9 definiert als kosmetisches Produkt.

11. Methode zur kosmetischen Behandlung von Comedonen, dadurch gekennzeichnet, daß man die kosmetischen Zusammensetzungen wie in Anspruch 9 definiert verwendet.

12. Verfahren zur Herstellung von kosmetischen Zusammensetzungen, die für die kosmetische Behandlung von Comedonen vorgesehen sind, dadurch gekennzeichnet, daß man als Wirkstoff mindestens eine der Verbindungen der Formel (I) wie in irgendeinem der Ansprüche 1 bis 3 definiert oder mindestens eines ihrer Salze, in allen möglichen isomeren, racemischen, enantiomeren und diastereoisomeren Formen, in eine für diese Verwendung bestimmte Form bringt.